# EUROPEAN PATENT APPLICATION

(11) **EP 1 872 826 A2**
(43) Date of publication of application: **02.01.2008**
(21) Application number: 07012674.3
(22) Date of filing: 28.06.2007
(51) Int. Cl.: A61N 1/32

(54) **Electrostimulator**

(30) Priority: 30.06.2006 IT NA20060082
(71) Applicant: Cappelletti, Renato, 80078 Pozzuoli (NA) (IT)
(72) Inventor: Cappelletti, Renato, 80078 Pozzuoli (NA) (IT)
(74) Representative: Barberi, Vittorio

(57) **Abstract**

Multi-channel electro-stimulator, provided with means for generating stimulation signals and with a plurality of couples of terminals destined to the connection of corresponding electrodes, characterised in that said means for generating signals (2A, 2B) separately or simultaneously produce a voltage and/or a current stimulation and that said couples of terminals (A, A'; B, B' ; C, C'; D, D' ; E, E'; F, F') are connected to one another so that the first terminal of each couple (A) is connected to the second terminal (A') of the same couple and to the second terminals (B', C', D', E', F') of the other couples.

## Description

The present invention relates to an electro-stimulator, in particular of multi-channel type.

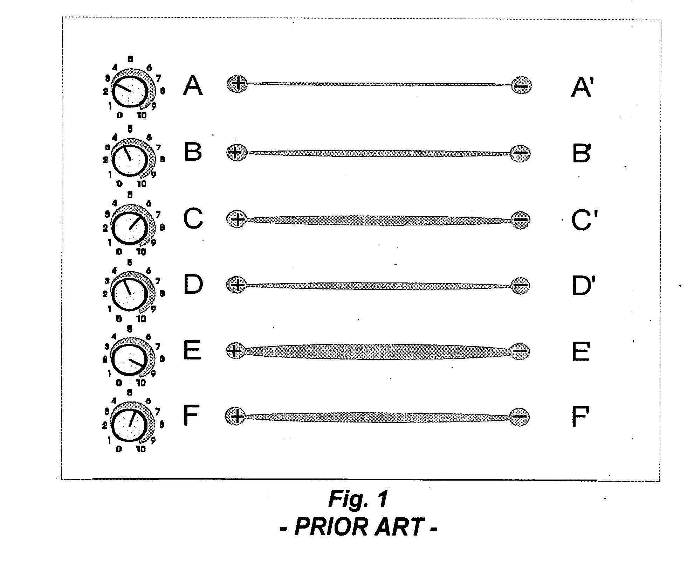

In their well-known embodiment, the multi-channel electro-stimulators consist of means for generating a signal which, on exit, are connected to a plurality of couples of terminals. According to the current constructive techniques of electro-stimulators and according to corresponding applicative methodologies, each channel or outlet is considered as autonomous and the circuit is closed only between the two stimulation terminals of an activated channel (galvanic isolation of the various outlets); in practice, there are no interferences between a couple of terminals and the other couples, independently from the number of channels used. The stimulation intensity is singularly adjustable in the different channels by means of a series of potentiometers. This adjustment theoretically allows the adaptation of the various stimulation channels to the resistive and/or capacitative variability of the single body zones submitted to electro-stimulation. Normally, in a typical multi-channel electro-stimulation treatment, each channel can be stimulated with a different intensity and each channel features a unidirectional electric vector (see Fig.1). This technology has been adopted to avoid the accumulation of electric charges in lower resistance zones during the simultaneous stimulation of a plurality of channels.

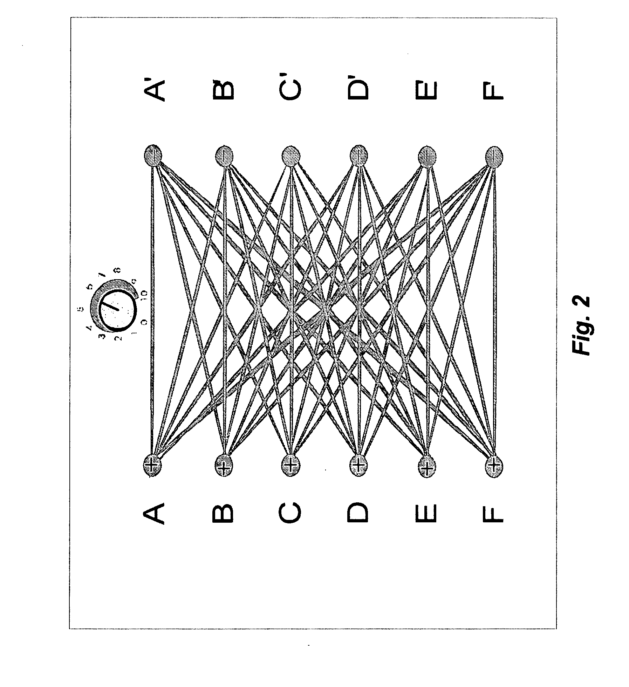

The electro-stimulator of the present invention, among its advantages, features a corresponding electric circuit which does not close between the respective couples of terminals, as normally happens in conventional multi-channel stimulators, but simultaneously among groups of terminals (see Fig.2). The whole system has been planned so as to avoid local resistive or capacitative interferences on the circuit closing and to guarantee a homogeneous distribution of the electric field by using a unified control system (by defining a homogeneous electric field). The electro-stimulator of the present invention uses two different stimulation systems, one is voltage-controlled, the other is current-controlled and they can be generated separately or simultaneously (so as to define an integrated electric field).

In practice, the invention is a new approach to electro-stimulation techniques thanks to the many interactions which take place between the set up magnetic field characterised by multiple electric vectors and the electro-stimulated zones. In other words, the multi-channel electro-stimulator of the present invention is able to determine an innovative, homogeneous and integrated electro-stimulation of a given zone. As the electric vectors generated by means of said electro-stimulator are not unidirectional, but multi-directional, the result of this is a more complex electro-stimulation, capable of involving the cutaneous, subcutaneous, neuromuscular and vascular systems. This feature offers the possibility of adopting new electro-stimulation methodologies, which can be used in various fields, from anthalgic therapy to aesthetic medicine and rehabilitation.

Moreover, the presence of multiple ionophoretic vectors determines an interesting interaction which takes place with possible ionized pharmacological substances injected into the subcutis. Multiple ionophoretic vectors are capable of interacting with ionized substances present along the propagation lines, to better distribute them locally or to facilitate their transmembrane passage along multidirectional migration lines as verified during the experimentation phase of the electro-stimulator. For example, the injection of a pharmacological substance into the subcutis (mesotherapy), may cause the formation of pomphus or local accumulations, which have a different substance density, from the injection point to the periphery of the pomphus itself. If the intervention on said pomphus takes place in the traditional way, that is to say with a univectorial electro-phoretic field, it is possible to obtain the migration and the consequent distribution of the ionized substances which are present locally along said vectors (linear ionophoresis). This distribution, however can only take place if the electrophoretic field acts on the pomphus. If , instead, a multivectorial electro-phoretic field is used (like in the present invention), the possibilities of an interaction between an electro-phoretic field and the pomphus increase, so as to determine a multilinear ionophoresis. Moreover, if the multivectorial electrophoretic static field changes into a variable field, the possibilities of an interaction increase remarkably, as this determines a tridimensional electro-phoretic field.

These and other advantages and characteristics of the invention will be best understood by anyone skilled in the art from a reading of the following description in conjunction with the attached drawings given as a practical exemplification of the invention, but not to be considered in a limitative sense, wherein:
- Fig 1 schematically shows a series of couples of electrodes which constitute the terminals of a multi-channel electro-stimulator of known type;
- Fig. 2 schematically shows a series of couples of electrodes which constitute the terminals of a multi-channel electro-stimulator according to the present invention;
- Fig. 3 schematically shows a possible embodiment of a multi-channel electro-stimulator according to the invention;
- Fig. 4 is a block scheme relative to a possible embodiment of a multi-channel electro-stimulator according to the invention.

With reference to the non-limitative examples of the enclosed drawings, the electro-stimulator of the invention will be now described. Said electro-stimulator is provided with means for generating signals and it features two different stimulation possibilities which can be used singularly or simultaneously, one of which is voltage-controlled (block 2A), while the other is current controlled (block 2B) having these two stimulation systems different prerogatives and aims, so as to represent two stimulation methodologies which are not alternative, but integrated.

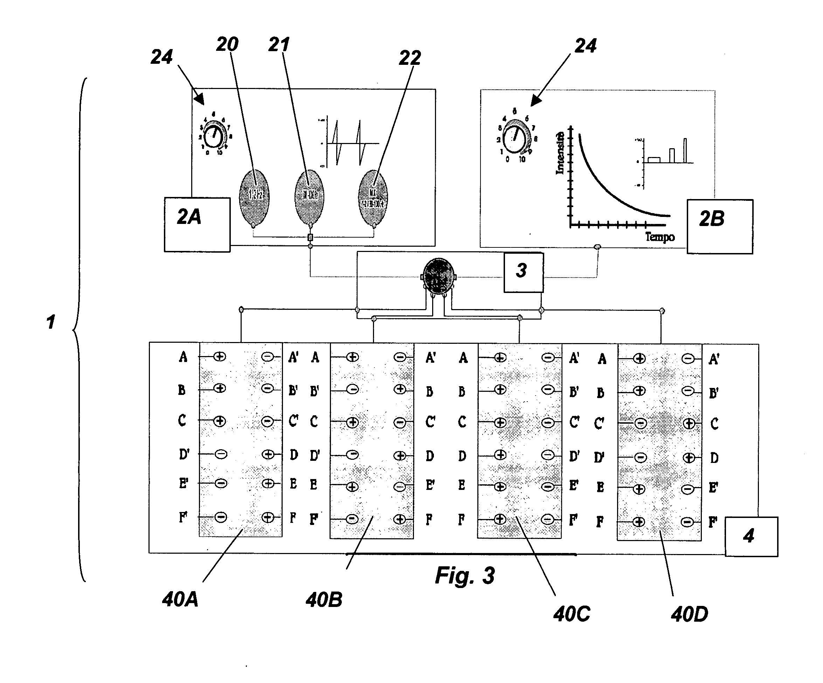

The voltage stimulation uses a biphasic signal, with frequency -dependent programs. The voltage stimulation exploits the biohumoral response of a living organism using special frequencies. As also experimentally verified, said stimulation tends to generate a sort of codified system, nearly an activation code, which can aimly interfere on the neurovegetative system with activation or inhibition phenomena of the sympathic or parasympathic system on the postural system with activation or inhibition phenomena, that is to say of relaxation or postural contraction and on the ascendant and/or descendant control systems (gate control theory). Three frequency types are preferably used to carry out said codification:
- low frequencies (1-2 Hz) schematically indicated by 20 in Fig.3;
- medium frequencies (80-100 Hz) schematically indicated by 21 in Fig.3;
- mixed frequencies with periodical alternation of medium and low frequencies, schematically indicated by 22 in Fig.3.

Indeed, it is possible to use specific frequencies as specific activation codes. The duty cycle of the signal used in the voltage stimulation is not fixed, but frequency-dependent, as it decreases when the frequency increases.

The current-stimulation (block 2B), instead ,generates a unidirectional, mono-phasic wave provided with an electrolitic and electrophoretic component. This feature can lead to risks of damages due to local iperpolarization of the galvanic component connected with said stimulation, if it is not adequately controlled. In order to avoid or to control said feature as adequately as possible, the electro-stimulator of the present invention is provided with a particular control system for the signal being used, which intervenes on the duty cycle of the signal, when the intensity of the delivered current varies (I/T system).

The present system is capable of adapting the duty cycle of the signal to the delivered intensity. The adjustment of the intensity-time relation allows the optimal use of all the potentialities offered by the intensity variation of the unidirectional stimulation, with no risks of damages due to local iperpolarization.

The unidirectional signal stimulation systems normally used feature a standard stimulation based on a prefixed duty cycle and on a variable intensity or, in some cases, they feature an adjustment based only on two points (cronaxy and rheobase). Said technology is not adequate because, as highlighted by studies on cronaxy and rheobase, electric stimulation has an optimum along a curve (function curve) which is intensity-time dependent. Therefore, if the time parameter is fixed (duty cycle), there will be a single effective intensity value, while all the other values will be over-or underestimated and vice-versa. Unlike the systems built up to now, the system of the present invention foresees a continuous and automatic adjustment (duty cycle) of the signal time in relation to the programmed intensity variation according to a preset function curve. Thanks to this system, whenever a parameter varies, also the other parameter varies automatically. It is also possible to foresee adjustment means 24 for the signals consisting of potentiometers or, for example, similar devices.

The aforementioned generated signals (voltage and /or current generated), singularly or combined with one another, are processed by a partition and commutation system, indicated by 3 and 4 in the drawings, which is capable of distributing an integrated and homogeneous signal on a plurality of channels/outputs. In particular, the device has been subdivided into a partition section 3 and in commutation section 4. A peculiarity of this partition is the possibility of closing the circuit no longer between couples of terminals, but among groups of terminals. The system is provided with a specific rating method and provides a multivectoral electric field with a uniform and controlled delivery of the employed currents, with no risk of o accumulation of charges on lower resistance electrodes. In fact, the system is controlled so as to render the local resistance and impedance variations unaffective on the signal distribution, thanks to a preset programme and to its dimensioning.

Partition device 3 is not static, because it is equipped with an adequate programme for the geometrical variation of terminals, capable of causing the system to close each time in a different way, when intervening manually or at predetermined times.

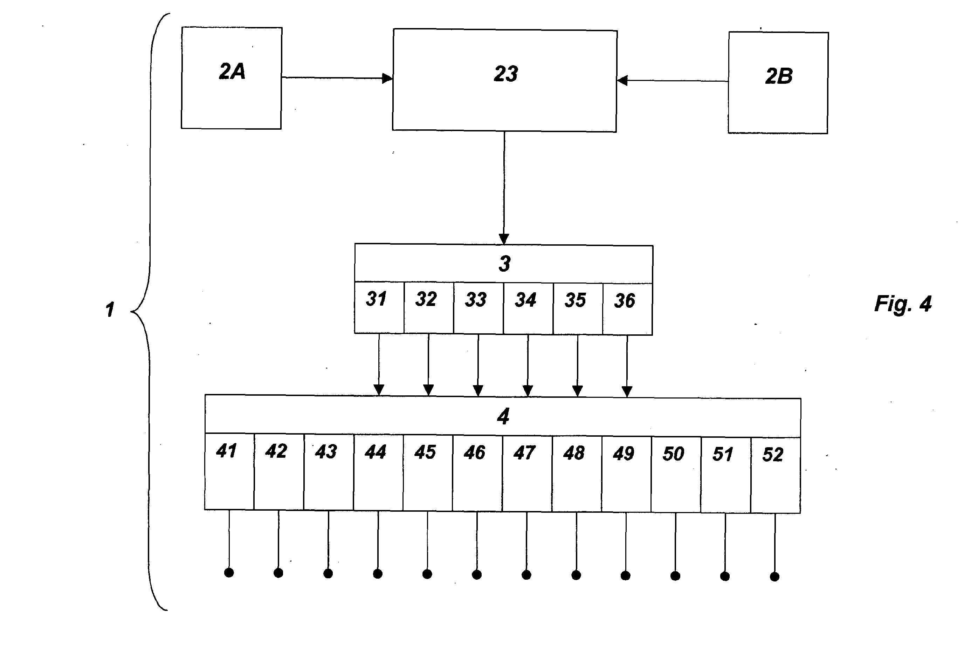

In other words, with reference to the scheme of Fig. 4, the two generated signals, one voltage controlled (coming from block 2A) the other current controlled (coming from block 2B) are conveyed by a mixing device 23 so as to reach said partition device 3 which is provided with six output channels numbered 31, 32, 33, 34, 35 and 36.

The time variation carried out by partition device 3 involves both the simple polarity inversion and a real geometrical redistribution of the stimulation system.

The so generated signals are capable of producing a homogeneous and integrated electric field characterized by multiple and variable electric vectors at predetermined times, capable of homogeneously and tridimensionally acting on the zone being treated and to electro-stimulate it.

Said signals are distributed, at the output, by means of a series of terminal-cables to be connected to electrode-needles or to cutaneous electrodes, with no obstacles due to problems of polarity.

In particular, the phase-commutation section 4, featuring said terminals on output which, in the example, are twelve and numbered from 41 to 52, is disposed downstream of partition section 3.

According to the kind of treatment to be carried out, terminals 41-52 can be connected to electrodes, plates, needles, tweezer-electrodes and so on, both of the external type (for example cutaneous plate electrodes) and of the internal type (electrode needles).

Fig. 2 shows the connection between the electrodes of the various couples. In the drawing, each of the six couples of terminals is marked by a couple of letters: A and A', B and B', C and C', D and D' E and E', F and F' .

Each first terminal of a couple is connected to the second terminal of its couple and to all the second terminals of the remaining couples.

For example, the first terminal A of the couple A-A' is connected to the second terminal A' of its own couple and to all the second terminals B', C', D', E', F' of the other couples.

As previously explained, to each terminal it is possible to connect an electrode which can be applied to the patient's body in order to carry out the corresponding treatment. The polarities of the terminals can be varied according to the treatment to be carried out.

In the example of Fig. 3, the 12 terminals presented on exit are marked by polarity symbols "+" and "-" and are shown in four possible embodiments which are marked with 40A, 40B, 40C and 40D. In practice, commutation section 4 can present terminals with differently distributed polarity on output according to the treatment to be carried out.

In other words, the terminals presented are in a fixed number (twelve in the example) but it is possible to present numerous configurations (four in the example).

By varying the activation modalities of the electrodes (keeping the distance of the electrodes constant among the electrodes disposed on the human body) it is possible to vary the depth of the corresponding electric field.

The example marked with 40C shows six positive polarities in correspondence of the left terminals (A, B, C, D, E, F) and six negative polarities in correspondence of the right terminals (A', B', C', D', E' , F').

In the example marked with 40C, the positive polarities are disposed in correspondence of the first three left terminals and of the last three right terminals (on the left: terminals A, B, C, D', E', F' and on the right: terminals A', B', C', D, E, F).

In the example marked with 40B, the positive polarities are alternately disposed one by one, on the left and on the right (on the left terminals A, B', C, D', E, F' and on the right terminals A', B, C', D, E', F).

Finally, in the example marked with 40D, the positive polarities are alternately disposed, two-by-two on the left and on the right (on the left: terminals A, B, C', D', E, F and on the right: terminals A', B', C, D, E', F').

During the experimentation phase of the electro-stimulator 1, the application of some theories has proved particularly interesting. In particular, in therapies which foresee the injection of a given substance into the human body there has been an increase in the action of the substance itself when the body part involved has been submitted to a treatment with the electro-stimulator of the present invention. Said therapies foresee the injection of a substance, which is homogeneously and uniformly distributed thanks to the intervention of the electro-stimulator. In practice, a sort of internal ionophoresis occurs and allows the use of only 50% of the medicine which is generally employed.

The control means of the elements described above and shown in the enclosed drawings are well-known to technicians, therefore they have not been described more in detail for further simplicity. Moreover, the execution details may equally vary as regards shape, size, disposition of the elements, kind of materials used within the solution idea that has been adopted and within the limits of the protection offered by the present patent.

## Claims

1. Multi-channel electro-stimulator, provided with means for generating stimulation signals and with a plurality of couples of terminals destined to the connection of corresponding electrodes, **characterised in that** said means for generating signals (2A, 2B) separately or simultaneously produce a voltage and/or a current stimulation and that said couples of terminals (A, A'; B, B'; C, C'; D, D'; E, E'; F, F') are connected to one another so that the first terminal of each couple (A) is connected to the second terminal (A') of the same couple and to the second terminals (B', C', D', E', F') of the other couples.

2. Electro-stimulator according to claim 1, **characterised in that** said means for generating stimulation signals (2A, 2B) are provided with at least three specific stimulation programs respectively for low frequencies ranging from 1 to 2 Hz, medium frequencies ranging from 80 to 100 Hz and mixed frequencies with an alternation of low and medium frequencies at predetermined times.

3. Electro-stimulator according to claim 1 **characterised in that** it consists of a voltage-stimulation system, with biphasic wave and variable duty cycle according to the frequency selected.

4. Electro-stimulator according to claim 1 **characterised in that** said means for generating the electro-stimulation signal generate a mono-directional rectangular wave current system with a variable duty cycle.

5. Electro-stimulator according to claim 4 **characterised in that** said means for generating the signal vary the amplitude of the duty cycle whenever the intensity varies, by simultaneously adapting the duty cycle of the signal being used to the delivered intensity.

6. Electro-stimulator according to claim 1 **characterised in that** it is provided with a non-static partition system (3,4) which is capable of intervening at predetermined times by varying the connection among terminals, so as to cause the corresponding circuit to close and producing a multivectorial electric field, that is to say by determining a special variation of the electric vectors present on the single terminals.

7. Multi-channel electro-stimulator provided with means for generating signals and with a plurality of couples of terminals destined to the connection of corresponding electrodes **characterised in that** said means (2A,2B) for generating signals separately or simultaneously produce a voltage and/or current stimulation.

8. Multi-channel electro-stimulator provided with means for generating signals and with a plurality of couples of terminals destined to the connection of corresponding electrodes **characterised in that** said couples of terminals (A, A'; B, B'; C, C'; D; D'; E, E'; F, F') are connected to one another so that the first terminal (A) of each couple is connected to the second terminal (A') of the same couple and to the second terminals (B', C', D', E', F') of the other couples.

9. Electro-stimulator according to one of the previous claims **characterised in that** it consists of a commutation and partition system of the signal (3, 4) disposed and acting between the means which generate the signal (2A, 2B) and the terminals to which the electrodes are to be connected.
